# EUROPEAN PATENT APPLICATION

(11) **EP 2 039 250 A1**
(43) Date of publication of application: **25.03.2009**
(21) Application number: 07116304.2
(22) Date of filing: 13.09.2007
(51) Int. Cl.: A01N 59/00, B01J 20/20, B01J 20/28

(54) **Composite powder with a high efficiency of releasing anions, and its attached substance and manufacturing method**

(71) Applicant: Yeh,, Jen-Taut, Taipei 106 (TW); Liang Haw Technology Co., Ltd., Taipei 103 (TW)
(72) Inventor: Yeh, Jen-Taut, 106 Taipei (TW); Yu, Li-Chun, 103 Taipei (TW); Chen, Kan-Nan, 103 Taipei (TW); Hsiung, Han-Hsing, 103 Taipei (TW)
(74) Representative: Lermer, Christoph

(57) **Abstract**

This invention discloses a composite powder with a high efficiency of releasing anions, and its attached substance and manufacturing method. The composite powder is made by mixing tourmaline powder and carbon-series powder in an optimal ratio, and the composite powder is blended with an attached substance (such as polymer foaming materials or chemical fiber materials) to form a product, such that the synergy of the composite powder results in high piezoelectricity, thermoelectricity and related physical properties to achieve the multifunctional effects of releasing anions, removing odors and suppressing germs.

## Description

### FIELD OF THE INVENTION

The present invention relates to a composite powder with a high efficiency of releasing anions, and its attached substance and manufacturing method, and a predetermined quantity of carbon-series powders is added to a tourmaline powder to achieve the multifunctional effects of releasing anions, removing odors and suppressing germs, and the manufacturing method is simple, easy and quick, which is applicable to mass productions and valuable to industrial applications.

### BACKGROUND OF THE INVENTION

Bamboo carbon is a porous material having a super large surface area and a very strong adhesion force. In the meantime, bamboo carbon also has the functions of removing odors and releasing anions, and its efficiency varies to a certain extent with the size of bamboo carbon particles. There is a natural mineral called tourmaline also having the function of releasing anions with the same limitation.

However, natural minerals used for releasing anions have been disclosed in issued patents and publications such as U.S. Pat. Nos. 6,192,949, 5,972,467, 5,967,207, 6,509,294, 6,449,990 and 6,475,513, wherein the main technical content of U.S. Pat. No. 6,192,949 discloses a method of manufacturing bamboo carbon by mixing salt based materials.

The main technical content of U.S. Pat. No. 5,972,467 discloses a method of manufacturing bamboo fiber slices, and the method splits a bamboo rod evenly into separate bamboo slices and heat treats the bamboo slices sufficiently to exterminate insect eggs in the bamboo slices; and applies resin and adhesive to the bamboo slices to form a bamboo cup vessel.

The main technical content of U.S. Pat. No. 5,967,207 discloses a method of fabricating bamboo slats for Venetian blinds, and the method splices the bamboo carbon slats, and then performs a bleaching oxidation, and finally manufactures the Venetian blinds.

The main technical content of U.S. Pat. No. 6,509,294 discloses a way for deodorizing and disinfecting germs for food preservation and construction materials by adding tourmaline and mixing adhesive to bamboo carbon to form a carrier, and then adding at least one of the chitosan, bamboo vinegar and conker acid to form an unwoven cloth.

The main technical content of U.S. Pat. No. 6,449,990 discloses a spherical purging apparatus of a washing machine, wherein a dirt and oil removing apparatus in the washing machine contains inorganic materials such as Bincloncharcoal and tourmaline mineral stone in a ratio approximately equal to 75:25 and 95:5, and the external surface of the purging apparatus is made of lightweight natural rubber or synthetic rubber.

The main technical content of U.S. Pat. No. 6,475,513 discloses a skin-care pouch including a sealed enclosure that contains carbides and water-absorbent shape-keeping agent for keeping skin moisture, removing cuticle and absorbing water.

The technical contents disclosed by the foregoing issued patents are different from the technical characteristics of the present invention, and the present invention provides a unique effect.

### SUMMARY OF THE INVENTION

In view of the foregoing shortcomings of the prior arts that involve a more complicated method and provide a limited effect, the inventor of the present invention based on years of experience in the related field to conduct extensive researches and experiments, and finally developed a composite powder with a high efficiency of releasing anions, and its attached substance and manufacturing method in accordance with the present invention.

It is a primary objective of the present invention to provide a composite powder with a high efficiency of releasing anions, and its attached substance and manufacturing method, wherein tourmaline powder and carbon-series powder are mixed with an optimal ratio to form a composite powder, and the composite powder is mixed together with an attached substance (such as a polymer foaming material or a chemical fiber material) to form a product, such that the product can provide a synergic effect, produce high piezoelectricity, thermoelectricity and related physical properties after the tourmaline powder and carbon-series powder are mixed, so as to achieve the multifunctional effects of releasing a large quantity of anions, removing odors and suppressing germs. In addition, the manufacturing method of the present invention is simple, easy and quick, which is suitable for mass production and useful to industrial application.

### BRIEF DESCRIPTION OF THE DRAWING

FIG. 1 is a schematic view of a composite powder sample analyzed by a scanning electron microscopy (SEM) in accordance with the present invention;
FIG. 2 shows an analysis of a ratio of composite powder contents versus an anion releasing concentration of the present invention;
FIG. 3 shows an analysis of an electric conductivity of a composite powder solution under different temperatures in accordance with the present invention;
FIG. 4 shows an analysis of average particle diameters distributed in a thermoplastic elastomer sample/thermoset elastomer sample when a composite powder content is adjusted in accordance with the present invention; and
FIG. 5 shows an analysis of an anion releasing concentration of a composite powder solution under different temperatures in accordance with the present invention.

### DETAILED DESCRIPTION OF THE PREFERRED EMBODIMENTS

To make it easier for our examiner to understand the objective of the invention, its structure, innovative features, and performance, we use a preferred embodiment together with the attached drawings for the detailed description of the invention.

The present invention provides a composite powder with a high efficiency of releasing anions, and its attached substance and manufacturing method. The composite powder of the invention comprises at least one mineral stone composite and other mineral powder, wherein the composite powder is formed by a primary composition of tourmaline powder added to a secondary composition of carbon-series powder in a predetermined ratio, so as to improve the electric field effect of the tourmaline granules and promote the multifunctional effects of releasing anions, removing odors, and suppressing germs. The tourmaline powder can be iron tourmaline, lithium tourmaline, manganese tourmaline, cesium tourmaline or magnesium tourmaline, and the carbon-series powder can be bamboo carbon, coconut carbon, activated carbon or charcoal.

In the present invention, the composite powder has a composite ratio of 1∼20: 99∼80 for the tourmaline powder and the carbon-series powder, an average powder particle diameter of 0.3µm∼1µm for the tourmaline powder, and an average powder particle diameter of 5µm∼20µm for the carbon-series powder.

The composite powder (tourmaline powder/carbon-series powder) is described in details as follows. From the analysis of carbon series (such as bamboo carbon) powder particles in the composite powder and elements on their surfaces, we know that the bamboo carbon is a porous substance with a wide distribution of holes and crevices therein, and the tiny holes of different sizes and diameters provide a very strong adhesion capability.

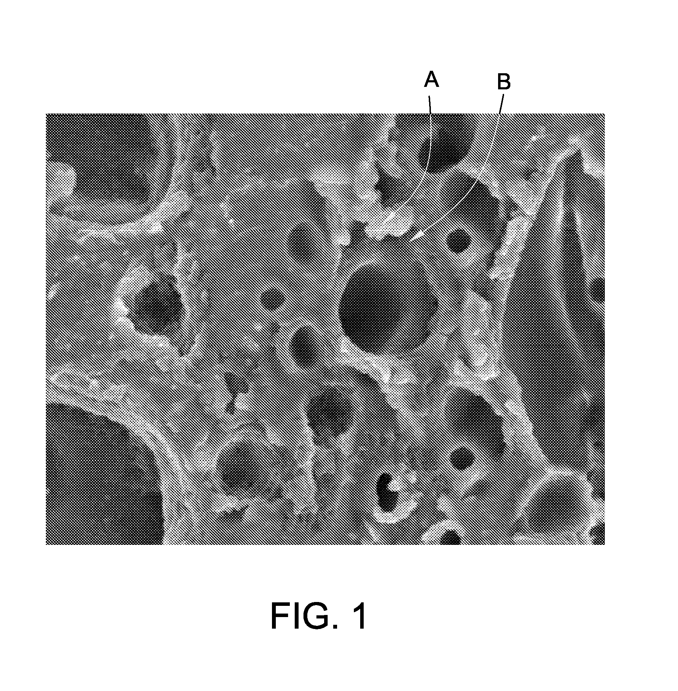

Referring to FIG. 1 for a schematic view of a composite powder sample analyzed by a scanning electron microscopy (SEM) in accordance with the present invention, tiny holes of different sizes are distributed on the surface of the bamboo carbon particles. The element composites at Points A and B on a surface of the bamboo carbon are different, and they are made of two different elements. The results of an energy dispersive X-ray (EDX) element analysis show that the tourmaline powder particles are mainly made of oxygen (O), aluminum (Al), silicon (Si), iron (Fe) and sodium (Na), wherein the oxygen (O), aluminum (Al), silicon (Si) and iron (Fe) exceeds 95wt% of the total of the tourmaline powder.

The bamboo carbon particles primarily contain carbon (C) element which exceeds 95 wt%, and it is noteworthy to point out that the tourmaline powder particles are filled into the holes and crevices of the bamboo carbon particles to form the "mixture".

Therefore, the bamboo carbon powder is added with the tourmaline powder with a predetermined ratio, and the tourmaline powder is filled into mesh structure of the bamboo carbon. Since the tourmaline powder has the piezoelectric and thermoelectric properties, and the bamboo carbon powder has a good electric conductivity, both powders are filled into a substrate to form a three-dimensional micro conductive network for providing an excellent synergic effect as well as promoting the anion releasing effect.

If the tourmaline powder and the bamboo carbon powder are mixed with a predetermined ratio, an average air anion releasing concentration measured in a static testing condition and at a temperature of 90°C can reach 1480 anions/cc. If the composite powder composed of the tourmaline powder and bamboo carbon powder is similarly mixed with the bamboo carbon powder in a predetermined ratio, its average air anion releasing concentration is 800-1480 anions/cc, which is higher than any composite powder mixed with other ratios, and it may be due to the thermoelectric property of the tourmaline powder. If the tourmaline powder and the bamboo carbon powder in the composite powder are mixed with a predetermined ratio, the tourmaline powder can be distributed uniformly in the holes of the bamboo carbon powder, so as to produce a thermoelectric/piezoelectric effect, and enhance the anion releasing performance as the temperature rises.

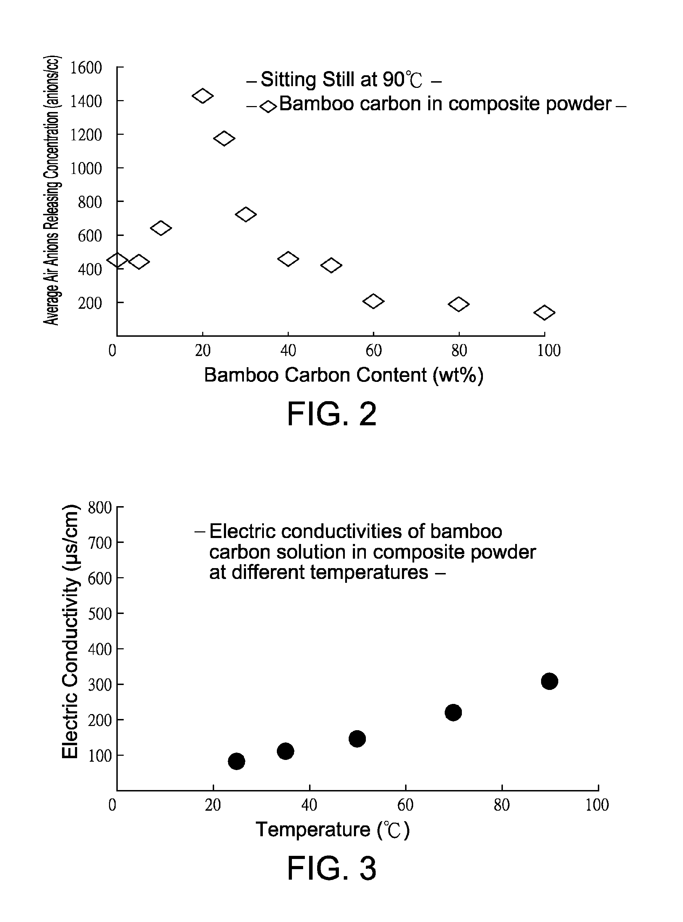

Referring to FIG. 2 for an analysis of a ratio of composite powder contents versus an anion releasing concentration of the present invention, an average air anion releasing concentration the composite powder increases accordingly with temperature. For instance, the tourmaline powder and the bamboo carbon powder in the composite powder are mixed in a predetermined ratio and measured in a static testing condition at 90°C, the air anion releasing concentration reaches its maximum 1480 anions/cc, which is approximately 2.8 times of the measured value obtained at a static testing condition at 35°C.

Referring to FIG. 3 for an analysis of an electric conductivity of a composite powder solution under different temperatures in accordance with the present invention, the bamboo carbon solution in the composite powder can effectively ionize water molecules into anions through the contact with water molecules to improve electric conductivity, and the electric conductivity tends to increase with temperature, and the average air anion releasing concentration of the foregoing composite powder sample also tends to increase with temperature. This conclusion further shows that the composite powder in liquid water or in air have a significant thermoelectric property.

In addition, the present invention further mixes the composite powder into an attached substance which can be a polymer material, and the polymer material generally refers to a thermoplastic and/or thermoset elastomer material (such as ethylene-propylene-non-conjugated diene rubber (EPDM)/polypropylene (PP) or EPDM, PP and PU, etc), and the polymer material can also be a product made of a foam material (such as polyurethane (PU), polyethylene (PE), polypropylene (PP) and polystyrene (PS), etc), and the elasticity and compressibility of the foam drive the tourmaline to maximize its piezoelectric performance and achieve the multifunctional effects of releasing a large quantity of anions, removing odors and suppressing germs.
The polymer material can be a chemical fiber material (such as polypropylene terephthalate (PET), polypropylene (PP), and nylon) for making textile products and achieving the multifunctional effects of releasing anions, removing odors and suppressing germs (as shown in Tables 1 and 2).

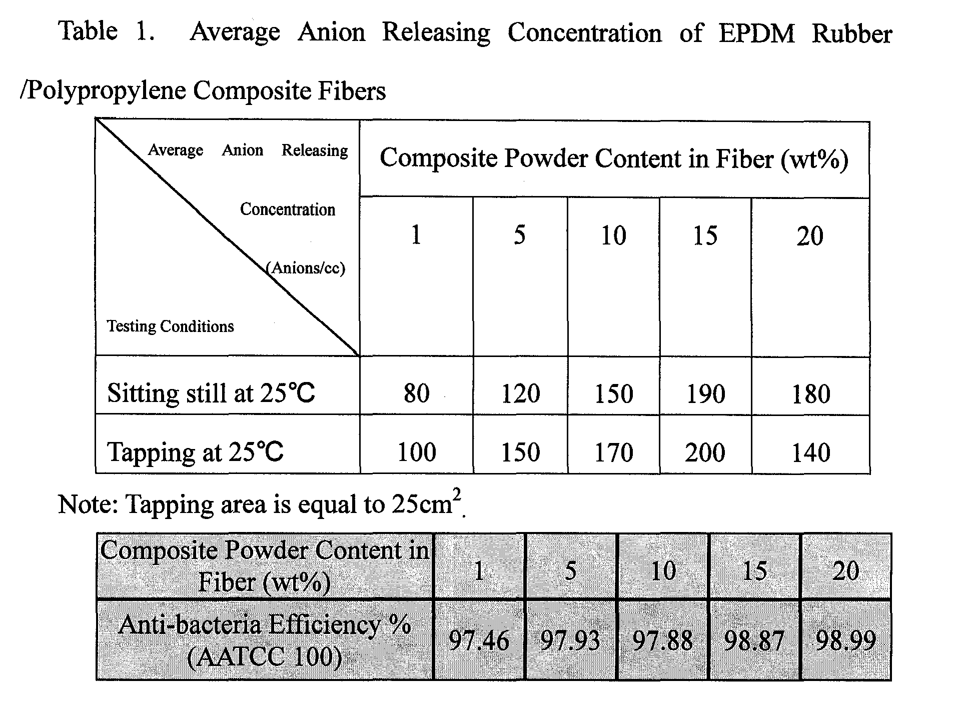

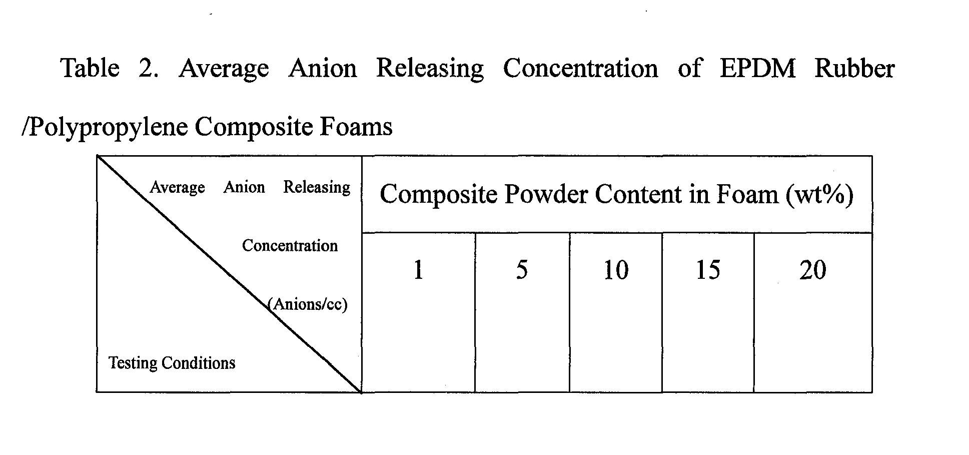

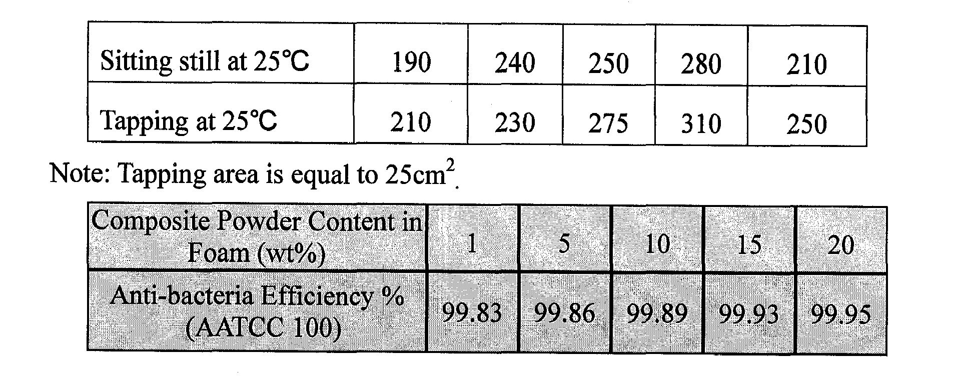

The composite powder (tourmaline powder/carbon-series powder) is mixed with a thermoplastic and/or thermoset elastomer material of the polymer material in the optimal average contents as follows.

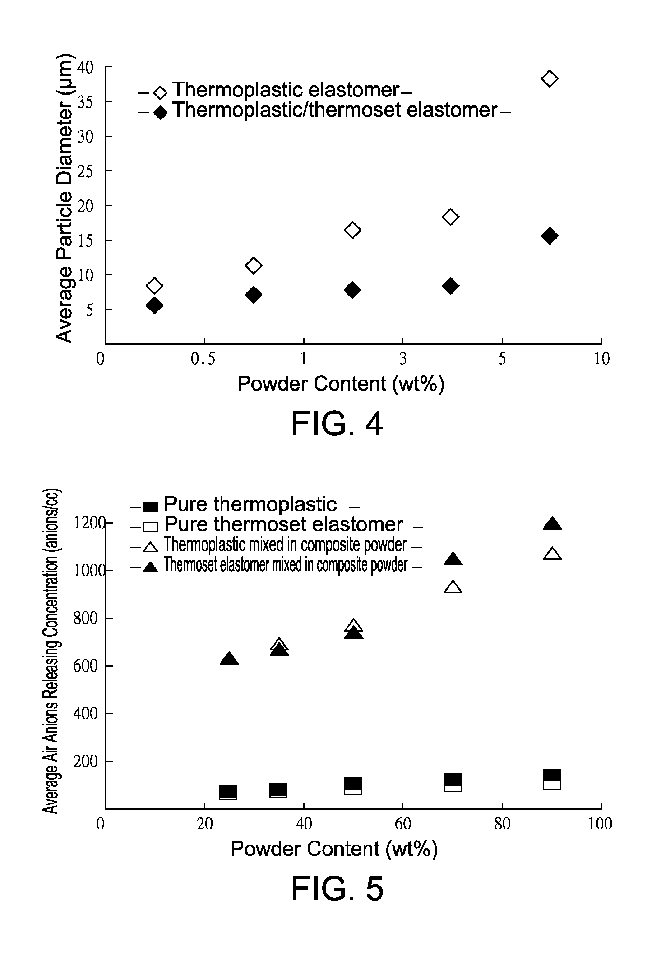

Referring to FIG. 4, when the composite powder content is increased from 0.5 wt% to 10 wt%, the average particle diameters distributed in the thermoplastic elastomer samples will be increased from 8.5µm to 38.8µm, which are approximately 2-13 times of the original particle diameter. If the composite powder content is increased from 0.5 wt% to 10 wt%, the average particle diameters distributed in the thermoplastic/thermoset elastomer sample will be increased approximately from 5.7µm to 15.7µm, which are approximately 1-6 times of the original particle diameter, and obviously smaller than the average particle diameter of the thermoplastic elastomer sample in equivalent conditions.

In addition, if the composite powder content in the thermoplastic elastomer sample is increased from 0.5 wt% to 5 wt%, the average particle diameters distributed in the thermoplastic elastomer samples will be increased slowly from 8.5µm to 18.6µm. If the composite powder content is increased from 5 wt% to 10 wt%, the average particle diameters distributed in the thermoplastic elastomer samples will be increased significantly and rapidly from 18.6µm to 38.8µm.

Similar trend occurs in the thermoplastic/thermoset elastomer sample; if the composite powder content is increased from 0.5 wt% to 5 wt%, the average particle diameters distributed in the thermoplastic/thermoset elastomer samples will be increase slowly from 5.7µm to 8.5µm; and if the composite powder content is increased from 5 wt% to 10 wt%, the average particle diameters distributed in the thermoplastic/thermoset elastomer samples will be increased significantly from 8.5µm to 15.7µm.

From the above results, we can infer that if the composite powder content is less than 5 wt% during a manufacturing process, the composite powders can be distributed better in the plastics of thermoplastic elastomer (such as PP) and thermoplastic/thermoset elastomer (such as EPDM/PP). However, if the composite powder content is greater than 5 wt%, an obvious agglomeration will occur.

The average air anion releasing concentrations of a pure thermoplastic/thermoset elastomer sample and a thermoplastic/thermoset elastomer sample containing a composite powder are measured at different testing conditions as described below.

Referring to FIG. 5, the average air anion releasing concentration of the pure thermoplastic/thermoset elastomer sample measured in the test condition at 25°C is 30 anions/cc only. After the composite powder is mixed into the pure thermoplastic/thermoset elastomer sample, it is obvious that the average air anion releasing concentration increases accordingly with the composite powder content, temperature and pressure. It is noteworthy to point out that if the content of composite powder is mixed to an optimal value, the average air anion releasing concentration will be maximized. For measurements taken at a still condition at 25°C, the average air anion releasing concentration will be approximately equal to 270 and 400 anions/cc, which is more than 6 times of the average anion releasing concentration of the pure thermoplastic/thermoset elastomer sample measured in equivalent conditions.

Since tourmaline powder has both thermoelectric and piezoelectric properties, therefore the average air anion releasing concentration of a thermoplastic/thermoset elastomer sample containing a composite powder mixed with a pure thermoplastic/thermoset elastomer sample is higher than the results obtained from a still condition at room temperature, when the temperature rises and the pressure changes. If the temperature exceeds 50°C, the evaporation of water near the thermoplastic/thermoset elastomer sample of the composite powder will speed up to favor the ionization of water in air by the tourmaline powder, and thus the average air anion releasing concentration will become higher.

However, the tourmaline powder has the piezoelectric effect, and thus the thermoplastic/thermoset elastomer sample containing tourmaline powders has a better anion releasing effect than the pure thermoplastic/thermoset elastomer sample. Furthermore, the bamboo carbon powder also has the anion releasing effect, and adding an appropriate quantity of bamboo carbon powder as a secondary composition can improve the electric field effect of the tourmaline powder, so that the tourmaline powder and the bamboo carbon powder can produce a synergic effect to enhance the anion releasing effect.

In addition, the composite powder of the invention is mixed with an attached substance, which is a thread or foam grade polymer material, wherein the polymer material refers to a thermoplastic and/or thermoset elastomer material and another fiber or foam material (such as including polyethylene (PE), polypropylene (PP), ethylene-propylene-non-conjugated diene rubber (EPDM), ethylene-vinyl acetate (EVA) and artificial rubber, such as styrene-butadiene rubber (SBR), neoprene and other polymer material) in form of a concentrate, and the desired mixing polymer material (such as nylon, polyester, polypropylene, polyethylene and polyurethane) is melted at a specific temperature and formed by a specific mixing method including spraying, mechanical mixing or gas/liquid fluid for mixing the composite with the polymer material. Further, the polymer material in form of a concentrate is formed into a linear fiber substance by compression and stretching energies, and the polymer material in form of a concentrate can be attached to a sheet structural substance formed by compression and stretching energies, and foamed and shaped at a specific temperature.

The products made of a foam material include exercise mats, decorative wallpapers, floor linings, carpet linings, insoles or mid-soles, protective pad, indoor temperature preserving materials, etc.

The polymer material can be a chemical fiber material (including an artificial fiber such as rayon, a synthetic fiber such as polyethylene (PE), polypropylene (PP), polyacrylonitrile (PAN), a nylon fiber, polypropylene terephthalate (PET) used for making textile products.

The aforementioned chemical fiber materials are applied in products including curtain fabrics, sofas, carpets, clothes, umbrella canopy fabrics, bed sheets, wall decoration fabrics, car seat covers and decorative linings, etc.

The manufacturing method of a composite powder in accordance with the present invention, at least one mineral stone composite and another mineral powder composite under a highly clean environment are used for producing a composite powder by a specific grinding energy technology, and the composite powder contains a primary composition of tourmaline powder added to a secondary composition of carbon-series powder in a specific ratio to achieve the anion releasing function of high piezoelectricity, thermoelectricity or related physical properties, wherein the specific grinding energy technologies include manufacturing method such as an object collision, an energy collision, a mechanical energy mixing, or another gas/liquid fluid mixing.

The composite powder of the invention can be mixed with a threat or foam grade polymer material (such as nylon, polyester, polypropylene, polyethylene and polyurethane) in form of a concentrate, and the desired mixing polymer material is melted at a specific temperature and produced by one of the specific mixing methods as described below:
(1) Mechanical mixing method for a thread grade polymer material: a polymer material (such as polypropylene terephthalate (PET), polypropylene (PP), and nylon) sample is mixed with a pre-mixed tourmaline and bamboo carbon composite powders in a specific ratio in a screw extrusion machine. The screw extrusion machine is operated at a temperature of 150-250°C and a rotation speed of 8-15Hz, and the polymer composite melted at high temperature is extruded by a screw rod and flow into a filament box, and then sprayed from a spinneret and cooled and stretched into artificial silk with an anion releasing effect.
(2) Concentrate manufacturing method for a foam grade polymer material:
   The polymer material in form of a concentrate mixes a thermoplastic (such as hydrogenated thermoplastic SBR, thermoplastic NBR, EPDM sample with a thermoset elastomer (such as amide, polyester, epoxy resin, urea formaldehyde, phenol formaldehyde) sample in an appropriate ratio, and the mixing ratio is approximately equal to 15/95-75/5, and 7%-20% of phenolic resin and 5%-10% of tin dichloride catalyst and premixed tourmaline and bamboo carbon composite powders in a screw extrusion machine. The composite material is attached onto a linear rod substance formed by extrusion and stretching energies (with a screw rod operated at a rotating speed of 5-10Hz and a temperature of 150-250°C), and solidified in a cooling water tank (at 5-10°C) to form a continuous linear composite polymer, and finally cut into particles by a rotary cutter of a granule cutting machine.

It is to be understood, however, that even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A composite powder with a high efficiency of releasing anions, comprising:
a composite powder, composed of at least one mineral stone composite and
other mineral powders, and formed by adding a tourmaline powder composition added with a carbon-series powder composition, and the tourmaline powder and the carbon-series powder being mixed in a ratio of 1∼20: 99∼80.

2. The composite powder with a high efficiency of releasing anions as recited in claim 1, wherein the tourmaline powder is made of iron tourmaline, lithium tourmaline, manganese tourmaline, cesium tourmaline or magnesium tourmaline, and the carbon-series powder is made of bamboo carbon, coconut carbon, activated carbon or charcoal.

3. The composite powder with a high efficiency of releasing anions as recited in claim 2, wherein the tourmaline powder has an average powder particle diameter of 0.3µm∼µm, and the carbon-series powder has an average powder particle diameter of 5µm∼20µm.

4. An attached substance of a composite powder with a high efficiency of releasing anions, comprising:
a composite powder, composed of at least one mineral stone composite and
other mineral powders, and having a primary composition of a tourmaline powder added with a secondary composition of carbon-series powder, and
the tourmaline powder and the carbon-series powder being mixed in a ratio of 1∼20: 99∼80; and
an attached substance, mixed together with the composite powder.

5. The attached substance of a composite powder with a high efficiency of releasing anions as recited in claim 4, wherein the attached substance is a polymer material selected from a thermoplastic and/or thermoset elastomer material, and another fiber or foam material.

6. The attached substance of a composite powder with a high efficiency of releasing anions as recited in claim 5, wherein the polymer material is a foam product made of a foam material.

7. The attached substance of a composite powder with a high efficiency of releasing anions as recited in claim 5, wherein the polymer material is a textile product made of a chemical fiber material.

8. The attached substance of a composite powder with a high efficiency of releasing anions as recited in claim 6, wherein the foam material is one selected from the collection of polyethylene (PE), polypropylene (PP), ethylene-propylene-non-conjugated diene rubber (EPDM), ethylene-vinyl acetate (EVA) and artificial rubber.

9. The attached substance of a composite powder with a high efficiency of releasing anions as recited in claim 6, wherein the foam material is applied to a product including an exercise mat, a decoration wallpaper, a floor lining, a carpet lining, an insole or mid-sole, a protective pad, and an indoor temperature preserving material.

10. The attached substance of a composite powder with a high efficiency of releasing anions as recited in claim 7, wherein the chemical fiber material includes an artificial fiber, a synthetic fiber, polyethylene (PE), polypropylene (PP), polyacrylonitrile (PAN), a nylon fiber and polypropylene terephthalate (PET).

11. The attached substance of a composite powder with a high efficiency of releasing anions as recited in claim 7, wherein the chemical fiber material is applied to a product including a curtain fabric, a sofa, a carpet, a cloth, an umbrella canopy fabric, a bed sheet, a wall decoration fabric, a car seat cover and a decorative lining.

12. A manufacturing method of a composite powder with a high efficiency of releasing anions, using a specific grinding energy technology to produce a composite powder from at least one mineral stone composite and other mineral powders, and the composite powder being made by a primary composition of tourmaline powder added with a secondary composition of carbon-series powder, and the tourmaline powder and the carbon-series powder being mixed in a ratio of 1∼20: 99∼80.

13. The manufacturing method of a composite powder with a high efficiency of releasing anions as recited in claim 12, wherein the specific grinding energy technology includes a manufacturing method adopting object collision, energy collision, mechanical energy mixing or other gas/liquid fluid mixing.

14. The manufacturing method of a composite powder with a high efficiency of releasing anions as recited in claim 12, wherein the composite powder is further mixed with a polymer material having a thread or foam grade condition, and formed into a concentrate, and the desired mixing polymer material is melted at a specific temperature condition and formed by a specific mixing method.

15. The manufacturing method of a composite powder with a high efficiency of releasing anions as recited in claim 14, wherein the polymer material in form of a concentrate is attached onto a linear fiber substance formed by compression and stretching energies.

16. The manufacturing method of a composite powder with a high efficiency of releasing anions as recited in claim 14, wherein the polymer material in form of a concentrate is attached onto a sheet structural substance formed by compression and stretching energies, and processed at a predetermined temperature to form a foam material.
